# EUROPEAN PATENT SPECIFICATION

(11) **EP 1 439 816 B1**
(45) Date of publication and mention of the grant of the patent: **15.10.2008**
(21) Application number: 02762332.1
(22) Date of filing: 06.07.2002
(51) Int. Cl.: A61Q 19/00, A61Q 19/10, A61Q 1/00, A61K 8/02, A61K 8/25, C01B 33/113

(54) **USE OF GRANULATES BASED ON PYROGENICALLY-PRODUCED SILICON DIOXIDE IN COSMETIC COMPOSITIONS**
VERWENDUNG VON GRANULATEN IN KOSMETISCHEN ZUSAMMENSETZUNGEN AUF BASIS VON PYROGENISCH HERGESTELLTEM SILIZIUMDIOXID
UTILISATION, DANS DES COMPOSITIONS COSMETIQUES, DE GRANULES A BASE DE DIOXYDE DE SILICIUM PREPARE DE FACON PYROGENE

(30) Priority: 30.10.2001 DE 10153077
(43) Date of publication of application: 28.07.2004
(73) Proprietor: Evonik Degussa GmbH, 45128 Essen (DE)
(72) Inventor: HASENZAHL, Steffen, 63454 Hanau (DE); RIEDEMANN, Heike, D-63776 Mömbris (DE); MEYER, Jürgen, D-63811 Stockstadt (DE); NEUGEBAUER, Peter, D-63071 Offenbach (DE)
(86) International application number: PCT/EP2002/007587
(87) International publication number: WO 2003/037287

(56) References cited:
- EP-A- 1 103 249
- EP-A- 1 172 095
- WO-A-01/30310
- GB-A- 1 093 139
- US-A- 4 000 317
- US-A- 4 312 845
- US-A- 5 711 797
- US-A- 5 776 240

## Description

The present invention relates to the use of granulates of pyrogenic silica in cosmetic compositions. In addition to various other effects, the granulates can perform the function of a carrier for cosmetic active ingredients and/or auxiliary substances.

It is known to use fumed (pyrogenically produced) silica in cosmetic compositions (see
US 5,711,797
WO 01/30310
GB 1,093,139
US 4,312,845
US 4,000,317
EP 1 103 249
EP 1 172 095).

Granules on basis of fumed silica are know for the use as catalyst support (see US 5,776,240).

The use of spherical silicon dioxide particles in cosmetic preparations is known. They are used, for example, in skin care and decorative cosmetics, to produce a smooth and velvety feeling of the skin ("ball bearing effect"); to produce the so-called "soft focus effect", which gives an even distribution of light falling on the skin and thus reduces the appearance of wrinkles and flaws in the skin; to improve the resistance of make up, by adsorbing the tallow produced by the skin or as carriers for cosmetic active ingredients, cosmetic oils and colourants.

US PS 4 837 011 discloses the use of spherical silicon dioxide particles with an average particle size of 6 to 20 µm in a cosmetic powder. The silicon dioxide particles used are obtained according to JP OS 61174103 by spraying a dispersion of silica sol and silica gel in a gas stream followed by drying. The silicon dioxide-containing powder is characterised by excellent adhesion to the skin and can be applied easily and smoothly.

EP OS 0 679 382 discloses spherical silicon dioxide particles with a preferred average particle size of 1 to 50 µm, which contain a UV-reflecting metal oxide and are coated with N-lauryl-L-lysine, and also cosmetic compositions containing these silicon dioxide particles. The cosmetics are easy to apply, make the skin feel soft and have good skin adhesion and water repellence. Both porous and non-porous silicon dioxide particles can be used, various methods being disclosed for the production of the porous particles. For example, fine liquid droplets of a silicate solution can be gelled with carbon dioxide in a non-polar organic solvent in the presence of a surfactant, washed and dried; fine droplets of a silicic acid ester can also be formed in a solvent, which are then gelled with ammonia or similar; or spherical silicon dioxide particles are produced by spray-drying silica sols. Non-porous spherical silicon dioxide particles can be obtained e.g. by tempering spherical or irregularly-shaped silicon dioxide particles in a gas stream at raised temperature.

WO OS 96/17583 relates to a cosmetic preparation, which contains spherical silicon dioxide particles with an average particle size of 3 to 16 µm. The silicon dioxide particles here serve on the one hand as carriers for moisture-containing agents, on the other to absorb excess tallow from the skin. No process for the production of silicon dioxide particles is disclosed.

DE OS 199 29 109 provides spherical silicon dioxide particles with a diameter of 50 nm to 50 µm and a coating of a metal oxide or a metal oxide and a colouring material. In addition to the use of these coated silicon dioxide particles as colour pigments in paints, printing inks and plastics, their use in cosmetic formulations is also disclosed. The coated silicon dioxide particles have excellent colouring power, good reproducibility of colouring and, in cosmetic applications, give the skin a soft and velvety feel.

DE OS 198 42 134 relates to similar spherical silicon dioxide particles with a diameter of less than 50 µm, which are coated either with titanium dioxide and silicon dioxide or with titanium dioxide and Iron(III)-oxide. These silicon dioxide particles are used in cosmetic formulations on the one hand to give the skin a natural appearance, but on the other to make wrinkles largely invisible. This is achieved by diffusing the reflected and transmitted light.

The spherical silicon dioxide particles from DE OS 199 29 109 and DE OS 198 42 134 are produced by hydrolysis of organic or inorganic silicon compounds in an emulsion process, as disclosed for example in DE OS 21 55 281, DE OS 26 10 852, GB OS 1 141 929 and EP OS 0 162 716.

Cosmetic exfoliating products to remove skin impurities and dead skin cells, which contain a particle-shaped material that can be rubbed away, with a particle size in the range 0.03 to 3 mm, are disclosed in WO OS 94/12151. The material that can be rubbed away may be for example agglomerates of precipitated silica with a primary particle size of 0.001 to 0.2 µm.

The disadvantage of all the silicon dioxide particles mentioned above, which are used in cosmetic peperations, is that they are laborious and costly to produce. Thus either expensive starting compounds such as silicic acid esters or silica sols are used, or silicates, which then often produce products of insufficient purity for the desired application - they often still contain considerable quantities of salts - so that laborious washing is required.

The object of the present invention is therefore to provide spherical silicon dioxide particles for use in cosmetic compositions, which do not have the stated disadvantages and also fulfil the exacting requirements of the cosmetics industry for purity and product safety.

The object is achieved by the use of a granulate based on pyrogenically-produced silicon dioxide in a cosmetic composition, whereby the granulate based on pyrogenically-produced silicon di-oxide has an average grain size of 10 to 120 µm and a BET-surface of 40 to 400 m²/g (determined to DIN 66 131 with nitrogen). The present invention also provides a cosmetic compositions that contains a granulate based on pyrogenically-produced silicon dioxide and conventional cosmetic constituents. The present invention further relates to an adsorbate consisting of a granulate based on pyrogenically-produced silicon dioxide and at least one other substance, selected from cosmetic active ingredients and auxiliary substances, and the production of such adsorbates.

More preferably, the silicon dioxide granulate also has the following physical-chemical reference data, determined as disclosed in EP PS 0 725 037:

| | |
|---|---|
| Pore volume: | 0.5 to 2.5 ml/g |
| Pore size distribution: | less than 5 % of the total pore volume has a pore diameter of less than 5 nm, the rest meso- and macro pores |
| pH-value: | 3.6 to 8.5 |
| Tamped density: | 220 to 700 g/l. |

A granuate suitable for the use according to the invention and the production thereof is disclosed for example in EP OS 0 727 037.

The granulate preferably contains meso- and macro pores, the volume of the meso pores constituting 10 to 80% of the total volume. The particle size distribution of the granulate is preferably 80 vol. % larger than 8 µm and 80 vol.% smaller than 96 µm. In a preferred embodiment of the invention, the proportion of pores smaller than 5 µm can be no more than 5 % in relation to the total pore volume.

The granulate used according to the invention can be produced, for example, by dispersing in water pyrogenically-produced silicon dioxide, preferably silicon dioxide produced from silicon tetrachloride by flame hydrolysis, spray drying it and then optionally tempering the granulate obtained at a temperature of 150 to 1,100°C for a period of 1 to 8 h.

The dispersion in water preferably has a concentration of silicon dioxide of 5 to 25 wt.%, more preferably 5 to approx. 19.9 wt.%. Spray drying can be carried out at a temperature of 200 to 600°C, using disk or nozzle atomisers. The granulate can be tempered both in a fixed bed, such as for example in chamber ovens, or in a fluid bed such as for example a rotary kiln.

The pyrogenic silicon dioxide used as a starting compound is produced by jetting a volatile silicon compound into an oxyhydrogen flame of hydrogen and air. In most cases, silicon tetrachloride is used. This substance hydrolyses under the influence of the water formed in the oxyhydrogen reaction to form silicon dioxide and hydrochloric acid.

After leaving the flame, the silicon dioxide enters a so-called coagulation zone, in which the silicon dioxide primary particles and primary aggregates agglomerate. The product present at this stage as a kind of aerosol, is separated in cyclones from the gaseous companion substances and then post-treated with moist hot air. This process reduces the residual hydrochloric acid content to less than 0.025%.

The granulates based on pyrogenically-produced silicon dioxide can also be silanised. The carbon content of the granulate is then preferably 0.3 to 15.0 wt.%. Halogen silanes, alkoxysilanes, silazanes and/or siloxanes can be used for silanation.

The following substances in particular can be used as halogen silanes:
Halogen organosilanes of the type X₃Si (CₙH₂ₙ₊₁)
   X = Cl, Br
   n = 1 - 20
Halogen organosilanes of the type X₂(R')Si(CₙH₂ₙ₊₁)
   X = Cl, Br
   R' = alkyl
   n = 1 - 20
Halogen organosilanes of the type X(R')₂Si(CₙH₂ₙ₊₁)
   X = Cl, Br
   R' = alkyl
   n = 1 - 20
Halogen organosilanes of the type X₃Si(CH₂) ₘ-R'
   X = Cl, Br
   m = 0.1 - 20
   R' = alkyl, aryl (e.g. -C₆H₅)
      -C₄F₉, -OCF₂-CHF-CF₃, -C6F₁₃, -O-CF₂-CHF₂
      -NH₂, -N₃, -SCN, -CH=CH₂,
      -OOC (CH₃) C=CH₂
      -OCH₂-CH (O) CH₂ 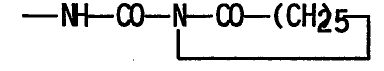 -NH-COO-CH₃, -NH-COO-CH₂-CH₃, -NH- (CH₂) ₃Si (OR) ₃
      -Sₓ-(CH₂)₃Si(OR)₃
Halogen organosilanes of the type (R)X₂Si(CH₂)ₘ-R'
   X = Cl, Br
   R = alkyl
   m = 0.1 - 20
   R' = alkyl, aryl (e.g. -C₆H₅) -C₄F₉, -OCF₂-CHF-CF₃, -C₆F₁₃, -O-CF₂-CHF₂
      -NH₂, -N₃, -SCN, -CH=CH₂,
      -OOC(CH₃)C = CH₂
      -OCH₂-CH(O)CH₂ 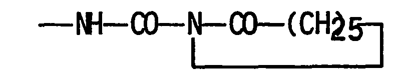 -NH-COO-CH₃, -NH-COO-CH₂-CH₃, -NH-(CH₂)₃
      Si(OR)₃-Sₓ-(CH₂)₃Si(OR)₃
Halogen organosilanes of the type (R)₂X Si (CH₂) ₘ-R'
   X = Cl, Br
   R = alkyl
   m = 0.1- 20
   R' = alkyl, aryl (e.g. -C₆H₅)
      -C₄F₉, -OCF₂-CHF-CF₃, -C₆F₁₃, -O-CF₂-CHF₂
      -NH₂, -N₃, -SCN, -CH=CH₂,
      -OOC(CH₃)C = CH₂
      -OCH₂-CH(O)CH₂ 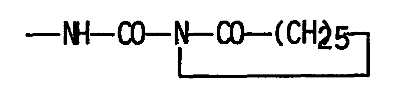 -NH-COO-CH₃, -NH-COO-CH₂-CH₃, -NH-(CH₂)₃Si(OR)₃
      -Sₓ-(CH₂)₃Si(OR)₃

The following substances can be uses in particular as alkoxysilanes:
Organosilanes of the type (RO)₃Si(CₙH₂ₙ₊₁)
   R = alkyl
   n = 1 - 20
Organosilanes of the type R'ₓ(RO)_{y}Si(CₙH₂ₙ₊₁)
   R = alkyl
   R' = alkyl
   n = 1 - 20
   x+y = 3
   x = 1.2
   y = 1.2
Organosilanes of the type (RO)₃Si(CH₂)ₘ R'
   R = alkyl
   m = 0.1 - 20
   R' = alkyl, aryl (e.g. -C₆H₅)
      -C₄F₉, OCF₂-CHF-CF₃, -C₆F₁₃, -O-CF₂-CHF₂
      -NH₂, -N₃, -SCN, -CH=CH₂,
      -OOC(CH₃)C = CH₂
      -OCH₂-CH(O)CH₂ 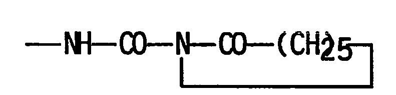 -NH-COO-CH₃, -NH-COO-CH₂-CH₃, -NH-(CH₂)₃Si(OR)₃
      -Sₓ-(CH₂)₃Si(OR)₃
Organosilanes of the type (R")ₓ(RO)_{y}Si(CH₂)ₘ-R'
   R" = alkyl
   x+y = 2
   x = 1.2
   y = 1.2
   R'= alkyl, aryl (e.g. -C₆H₅)
      -C₄F₉, -OCF₂-CHF-CF₃, -C₆F₁₃, -O-CF₂-CHF₂
      -NH₂, -N₃, -SCN, -CH=CH₂,
      -OOC(CH₃)C = CH₂
      -OCH₂-CH(O)CH₂ 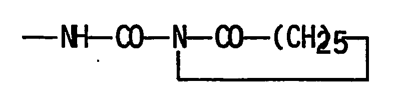 -NH-COO-CH₃, -NH-COO-CH₂-CH₃, -NH-(CH₂)₃Si(OR)₃
      -Sₓ-(CH₂)₃Si(OR)₃

Silane Si 108 [(CH₃O)₃-Si-C₈H₁₇] trimethoxyoctylsilane is preferably used as a silanising agent.

The following substances in particular can be used as silazanes:
Silazanes of the type: 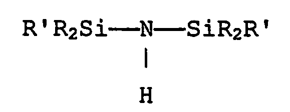
R = alkyl
R' = alkyl, vinyl
and also Hexamethyldisilazane, for example.

The following substances in particular can be used as siloxanes:
Cyclic polysiloxanes of the type D 3, D 4, D 5, e.g. octamethylcyclotetrasiloxane = D 4 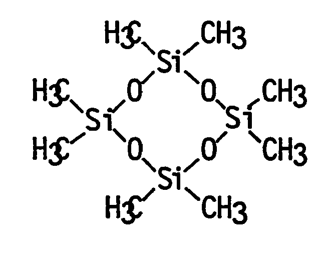 polysiloxanes e.g. silicon oils of the type: 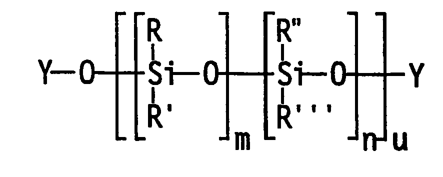
   R = alkyl, aryl, (CH₂)ₙ - NH₂, H
   R' = alkyl, aryl, (CH₂)ₙ - NH₂, H
   R" = alkyl, aryl, (CH₂)ₙ - NH₂, H
   R"' = alkyl, aryl, (CH₂)ₙ - NH₂, H
   Y = CH₃, H, CₙH₂ₙ₊₁ with n=1-20
   Y = Si(CH₃)₃, Si(CH₃)₂H
      Si(CH₃)₂OH, Si(CH₃)₂(OCH₃)
      Si(CH₃)₂(CₙH₂ₙ₊₁) with n=1-20
   m = 0,1,2,3,...∞
   n = 0,1,2,3,...∞
   u = 0,1, 2, 3, ... ∞

Silanation can be carried out by spraying the granulate with a silanising agent, which can optionally be dissolved in an organic solvent, such as for example ethanol, and then thermally treating the mixture at a temperature of 105 to 400°C for a period of 1 to 6 h.

An alternative method of silanising granulates can be used, in which the granulate is treated with the silanising agent in vapour form and then the mixture is thermally treated at a temperature of 200 to 800°C for a period of 0.5 to 6 h. The thermal treatment can take place in a protective gas, such as for example nitrogen.

Silanation can be carried out in heatable mixers and driers with spray devices, continuously or in batches. Suitable devices may be, for example: plough mixers, disk-or fluid bed-driers.

By varying the substances used, the spraying, tempering and silanation conditions, the physical-chemical parameters of the granulates, such as specific surface, the particle size distribution, the pore volume, the tamped density and the silanol group concentration, pore distribution and pH value can be changed within the given limits.

The granulates of pyrogenic silicon dioxide can be used according to the invention in cosmetic compositions of any consistency, e.g. in powders, liquids, foams, sprays, gels, creams, salves, pastes, sticks or tablets. Accordingly, the cosmetic compositions may be single- or multiphase systems such as for example emulsions, suspensions or aerosols.

The cosmetic composition may be, for example, a soap; a synthetic "soapless" soap; a liquid washing or shower preparation; a bath additive; a make-up remover; an exfoliating preparation; a skin cream; a skin lotion; a face mask; a footcare product; a sun protection product; a skin tanning product; a de-pigmenting product; an insect repellent; a wet-shave product, such as a stick, cream, gel or foam; a pre-shave product; an after-shave care product; a depilatory product; a toothpaste; a hair shampoo; a hair care product, such as a hair mask, a rinse or a conditioner; a permanent wave product; a smoothing product, a hair styling product, such as a setting lotion, a hair spray, a hair lacquer, a hair gel or a hair wax; a hair colourant, such as a bleaching product, a hair colouring product, a tint or a colour fixer; a deodorant or an anti-perspirant, such as a stick, roll-on, lotion, powder or spray; a face make-up, such as a tinted day cream, a cream-to-powder foundation, a face powder, a cream foundation or a blusher; an eye make up, such as an eyeshadow, a mascara, a kohl pencil, an eyeliner or an eyebrow pencil; a lip care product; a decorative lip care product, such as a lipstick, a lip gloss or a lipliner pencil; a nail care product, such as a nail polish, a nail polish remover, a cuticle remover, a nail hardener or a nail care cream.

The present invention also provides a cosmetic composition, containing the previously-defined silicon dioxide granulates and at least one constituent selected from absorbents, astringents, antimicrobial substances, antioxidants, anti-perspirants, anti-foam agents, anti-dandruff active ingredients, antistatic agents, binders, biological additives, bleaching agents, chelating agents, deodorants, emollients, emulsifiers, emulsion stabilisers, depilatory agents, colours, moisture-containing agents, film formers, perfumes, flavourings, hair colourants, preservatives, anti-corrosion agents, cosmetic oils, solvents, mouth care substances, oxidation agents, vegetable constituents, buffering agents, reducing agents, abrasives, detergents, propellent, opacity agents, UV filters and absorbers, denaturing agents, viscosity regulators and vitamins, whereby the granulate based on pyrogenically-produced silicon di-oxide preferably has an average grain size of 10 to 120 µm and a BET-surface of 40 to 400 m²/g (determined to DIN 66 131 with nitrogen)

Depending on the cosmetic composition, in which the silicon dioxide granulates are used, they may have various functions. They serve for example, to improve the feel of the skin (ball bearing effect), adhesion to the skin and ease of application. Furthermore, the long-term stability of decorative cosmetics such as make-up, is improved by the adsorption of skin tallow and oil. Also in decorative cosmetics, they improve the appearance of wrinkles by means of optimum, even distribution of light. In skin and hair cleansing products, the silicon dioxide granulates can act as abrasives.. They are also suitable for concealing or absorbing characteristic or even unpleasant odours of cosmetic consituents, which could otherwise not be used. A further function is the fixing, or slow and controlled release, of highly volatile substances, e.g. essential oils, aromas and perfumes. In many cosmetic compositions they also act as fillers. Hydrophobic silicon dioxide granulates i.e. silanised granulates., are particularly suitable for the production of waterproof cosmetics.

However, the silicon dioxide granulates used according to the invention preferably act as carriers for cosmetic active ingredients and/or auxiliary substances. The present invention thus also relates to an adsorbate of the silicon dioxide granulate mentioned previously and at least one of these substances whereby the granulate based on pyrogenically-produced silicon di-oxide preferably has an average grain size of 10 to 120 µm and a BET-surface of 40 to 400 m²/g (determined to DIN 66 131 with nitrogen)

The expression "adsorbate", as used here, encompasses not only the adsorption of a substance on the surface of the silicon dioxide, but also in the pores, and the "insertion" into the voids between the grains. "Adsorbate" can also mean that silicon dioxide granulate or fragments thereof, coats solid particles or liquid droplets of the substance. In the latter case, the attractive forces between the particles or droplets are reduced and, for example, the flow behaviour is improved or droplets are prevented from flowing together.

Examples of cosmetic active ingredients and auxiliary substances are the constituents of a cosmetic composition mentioned previously.

A cosmetic active ingredient according to this invention is deemed, as defined by Umbach (1995), to mean a substance in cosmetic preparations, which, under application conditions, has a physical, physical/chemical, chemical, biochemical and/or subject-related action, influencing inter alia the physiology and/or function of the skin or mucous membrane and their appendages, as well as the teeth, but excluding any significant effect on the organism. Examples of cosmetic active ingredients that can be adsorbed onto the silicon dioxide granules are vitamins; moisture-containing agents such as polyalcohols, ceramides and compounds similar to ceramides; physical and chemical UV filters and astringents.

Of the cosmetic auxiliary substances, cosmetic oils, perfumes, flavourings or colours are preferably adsorbed onto the silicon dioxide granulate. The perfumes and flavourings may be either of natural, i.e. vegetable or animal, or synthetic, i.e. fully- and semi-synthetic, origin.

Examples of vegetable perfumes are essential oils and resinoids. Animal perfumes include e.g. musk, civet, castoreum and ambergris. The fully-synthetic perfumes include both those having a natural equivalent and purely invented compositions. Semi-synthetic perfumes are understood to be those isolated from natural perfumes and then chemically altered.

The colourants can also be natural or synthetic; they may be organic or inorganic compounds.

The quantity ratio of substance to silicon dioxide granulate in the adsorbate may be selected at will, depending on the properties of the substance and the requirements of the end product. However, 0.001 to 200 g substance per 100 g silicon dioxide granulate is preferably used, and in particular 10 to 150 g.

An example of a process for the production of the adsorbate according to the invention comprises:
(a) melting of the substance(es) to be adsorbed, selected from cosmetic active ingredients and auxiliary substances, or distribution, i.e. dissolution, suspension or emulsification of these substances in a solvent;
(b) mixing of the granulate based en pyrogenically-produced silicon dioxide with the mixture from step (a); and
(c) optionally, removal of the solvent.

"Solvents" also include mixtures of several different solvents. It is also understood that substances that are liquid at room temperature can be subjected to the mixing in step (b) without any prior processing, as in this case the "melting process" has already taken place. The mixing step (b) can take place either by adding the mixture from step (a) to the silicon dioxide granulate, e.g. by spraying, or vice-versa. In both cases, addition can take place in one or several portions. The mixing time in step (b) depends primarily on the adsorption behaviour of the substance to be adsorbed on the silica surface. If a solvent is present, step (a) and step (b) are carried out at a temperature between the freezing and boiling point of the solvent. The optional excess solvent is removed in step (c), preferably at increased temperature and/or reduced pressure.

The removal of the solvent in step (c) can also be carried out either by spray- or fluid-bed drying, in which case the moulding process takes place simultaneously. Accordingly, the moulding process for a granulate-containing melt may be extrusion.

The advantages of the granulates of pyrogenic silicon dioxide used according to the invention are firstly that they are simple and inexpensive to produce and secondly that they are extremely pure.

The invention will now be explained in more detail with the aid of examples.

Reference example: Production of a granulate based on'pyrogenically-produced silicon dioxide.

The pyrogenically-produced silicon dioxide AEROSIL 90, commercially available from Degussa AG, is used as a starting compound.

The pyrogenically-produced silicon dioxide is dispersed in fully-desalinated water. A dispersing unit is used for this, which operates by the rotor/stator principle. The suspension formed is spray-dried. The finished product is separated by filter or cyclone. The spray granulate is tempered in a muffle kiln.

The production parameters are given in Table 1.

**Table 1**

| Starting-SiO₂ | AEROSIL 90 |
|---|---|
| Spray drying data | |
| Quantity H_{2O} (kg) | 100 |
| Quantity SiO₂ (kg) | 1.5 |
| Atomisation with | single-fluid nozzle |
| Operating temperature (°C) | 358 |
| Outlet air temperature (°C) | 105 |

| Separation | filter |
|---|---|
| Physical/Chemical Data | |
| BET-Surface (m²/g) | 87 |
| Grain size d⁵⁰µm) | 25 |
| Tamped volume (g/l) | 258 |
| pH-value | 4.7 |
| Carbon content % | - |

### Example 1: Tinted day cream with AEROSIL 90 granulate

A tinted day cream was produced according to the recipe in Table 2 and the method that follows, using 10 wt.% of the granulate produced in the reference example from AEROSIL 90.

**Table 2**

| | Constituent | INCI-Name | Wt.% | 2.00g |
|---|---|---|---|---|
| A | AEROSIL 90 granulate | Silica | 10.00 | 20.00 |
| | Veegum HV (Vanderbilt) | Magnesium Aluminium Silicate | 1.00 | 2.00 |
| | Euxyl K 400 (Schül ke+Mayer) | Methyldibromo Glu taronitrile, Pheno-xyethanol | 0.10 | 0.20 |
| | Sorbitol F liquid 1.02993 (Caelo) | Sorbitol | 3.00 | 6.00 |
| | Water | Aqua (Water) | 48.44 | 96.88 |
| B | Methyl-4-hydroxy-benzoate 1.30174 (Merck) | Methylparaben | 0.18 | 0.36 |
| | Propyl-4-hydroxybenzoate 1.30173 (Merck) | Propylparaben | 0.08 | 0.16 |
| | 1,2-Propanediol 1.30140 (Merck) | Propylene Glycol | 3.00 | 6.00 |
| C | Arlacel 165 (Uniqema) | Glyceryl Stearate, PEG-100 Stearate | 5.00 | 10.00 |
| | Lanette O (Caelo / Hen kel) | Cetearyl Alcohol | 1.50 | 3.00 |
| | Tegosoft CT (Gold schmidt) | Caprylic Capric Triglyceride | 7.00 | 14.00 |
| | Shea Butter (Karlshamns) | Butyrospermum Par kii (Shea Butter) | 2.00 | 4.00 |
| | Tegosoft CI (Cetiol SN) (Goldschmidt) | Cetearyl Isononanoate | 7.00 | 14.00 |
| | Eutanol G (Caelo / Hen kel) | Octyldodecanol | 7.50 | 15.00 |
| | Emulgade PL 68/50 (Hen kel) | Cetearyl Glucoside, Cetearyl Alcohol | 2.00 | 4.00 |
| D | Perfume | Parfum | 0.20 | 0.40 |
| | Dow Corning 345 (Dow Corning) | Cyclomethicone | 2.00 | 4.00 |
| | Citric acid (Merck) | Citric Acid | | |
| | | | 100.00 | 200.00 |

### Method:

The AEROSIL 90 granulate and the other components of phase A were added to the aqueous dispersion of Veegum in water whilst stirring. The resulting mixture was then heated to 80°C. The components of phase B were also mixed and heated until a clear mixture formed. Phase B was then added to phase A whilst stirring. The components of phase C were also mixed, heated to 80°C and quickly added to the mixed phases A and B. The resulting mixture was homogenised for one minute. It was allowed to cool to 40°C whilst stirring (blade mixer 500 min⁻¹) and phase D was then added.

The cream thus produced gave a pleasent feeling to the skin and could be applied to the skin easily. Viscosity (Brookfield, Speed 01, T-E Spindle, 26°C): 1545 mPa·s

### Example 2: Day cream containing vitamin E

50.0 g of the granulate produced in the reference example from AEROSIL 90 were placed into a tall 600 ml beaker and 50.0 g vitamin E acetate (BASF) were added in portions with a spatula. The granulate quickly absorbed the oily liquid, did not produce dust and did not become electrostatically charged. The entire quantity of vitamin E acetate was processed within ten minutes. The dry mixture was then sifted through a screen with a mesh width of 0.75 mm and allowed to stand overnight.

The flow mark and discharge cone height of the powdery adsorbate were then determined as described in the pigments literature series, No. 31 "AEROSIL for the improvement of the flow behaviour of powdery substances" from Degussa AG, bulk and tamped density were determined according to DIN standard 66131.

**Table 3: Characterisation of the vitamin E acetate adsorbate**

| | |
|---|---|
| Flow mark | 1 |
| Discharge cone height (cm) | 1.35 |
| Bulk density (g/l) | 431 |
| Tamped density (g/l) | 500 |

A cream analogous to example 1 was produced with the powdery adsorbate thus produced, using 10 wt.% of the vitamin E acetate adsorbate instead of 10 wt.% of the pure AEROSIL 90 granulate.

The cream thus produced gave a pleasant feeling to the skin and was easy to apply to the skin. Viscosity (Brookfield, Speed 01, T-E Spindle, 26.°C): 13600 mPa·s.

### Example 3: Cream-to-powder foundation

A cream-to-powder foundation was produced according to the recipe from Table 4 and the instructions that follow, using 10 wt.% of the granulate produced in the reference example from AEROSIL 90.

**Table 4**

| | Constituents | INCI-Name | wt.% |
|---|---|---|---|
| A | Tegosoft OP (Gold schmidt) | Octyl Palmitate | 29 |
| | Amerchol L-101 (Amerchol Corp.) | Mineral Oil, Lanolin Al cohol | 2.5 |
| | Carnauba wax (Caelo) | Carnauba | 1.3 |
| | Paracera W80 (Paramelt) | Ceresin | 2.0 |
| | Tegosoft CH (Gold schmidt) | Hydrogenated Castor Oil | 3.5 |
| | Dow Corning 556 (Dow Corning) | Phenyl Trimethicone | 9.5 |
| | Arlacel 83 (Uniqema) | Sorbitan Sesquioleate | 0.5 |
| | DL-alpha-Tocopherolacetate 5.00952 (Merck) | Tocopheryl acetate | 0.5 |
| | Propyl-4-hydroxybenzoate 1.3017.3 (Merck) | Propylparaben | 0.2 |
| | Oxynex K liquid 1.08324 (Merck) | PEG-8,Tocopherol,Ascorbyl Palmitat, Ascorbic Acid; Citric Acid | 0.1 |
| B | AEROSIL 90 granulate | Silica | 10.0 |
| | Amihope LL (Leh mann+Voss/ Ajinomoto) | Lauroyl Lysine | 1.5 |
| | Dry Flow PC (National Starch + Chemical) | Aluminium Starch, Octe nylsuccinate | 14.4 |
| | Extender W 1.17311 (Fa. Merck) | Mica, CI 77891 (Titanium Dioxide) | 12,0 |
| | Microna Matte Yellow 1.17436 (Merck) | Mica, CI77492 (Iron Oxi des) | 4.8 |
| | Microna Matte Black 1.17437 (Merck) | Mica, CI 77499 (Iron Di oxide) | 0.6 |
| | Microna Matte Red 1.17435 (Merck) | Mica, CI 77491 (Iron Oxi de) | 0.8 |
| | Microna Matte Orange 1.17449 (Merck) | Mica, CI 77491 (Iron Oxi de) | 0.4 |
| | Talk 1.08070 (Merck) | Talc | 6.4 |
| | | | 100 |

### Method:

The components of Phase A were mixed with each other and heated to 85° C whilst stirring producing a clear, liquid phase. The components of Phase B were also mixed and added to the molten Phase A whilst stirring. The liquid mixture was then poured into a mould at 80° C and left there to cool.

After cooling to room temperature, a solid, soft product was obtained that can be applied to the skin easily witch a sponge or brush. Resistance and adhesion to the skin were very good.

## Claims

1. Use of a granulate based on pyrogenically-produced silicon dioxide in a cosmetic compositions
**characterised in that**
the granulate has an average grain diameter of 10 to 120 µm and a BET-surface of 40 to 400 m²/g (determined to DIN 66 131 with nitrogen).

2. Use according to claim 1,
**characterised in that**
the cosmetic composition is in the form of a powder, liquid, foam, spray, gel, cream, salve, paste, stick or tablet.

3. Use according to one of claims 1, **characterised in that**
the cosmetic composition is a soap, synthetic "soapless" soap, liquid washing or shower preparation, bath additive, make-up remover, exfoliating product, skin cream, skin lotion, face mask, footcare product, sun protection cream, skin tanning product, de-pigmenting product, insect repellent, wet-shave product, pre-shave product, after-shave care product, depilatory product, toothpaste, hair shampoo, hair care product, permanent wave product, smoothing product; hair styling product, hair colouring product, deodorant, anti-perspirant, face make-up, eye make-up, lip care product, decorative lip care product or nail care product.

4. Use according to claim 3,
**characterised in that**
the wet-shave product is a stick, cream, gel, or foam;
the hair care product is a hair mask, rinse or conditioner;
the hair styling product is a setting lotion, hairspray, hair lacquer, hair gel or hair wax;
the hair colouring product is a bleaching agent, a hair colourant, a tint or a colour fixing agent;
the deodorant or anti-perspirant is a stick, roll-on, lotion, powder or spray;
the face make-up is a tinted day cream, a cream-to-powder foundation, a face powder, a cream foundation or a blusher;
the eye make-up is an eyeshadow, mascara, kohl pencil, eyeliner or eyebrow pencil;
the decorative lip care product is a lipstick, lipgloss or lipliner pencil and
the nail care product is a nail polish, nail polish remover, cuticle remover, nail hardener or a nail care cream.

5. Use according to one of claims 1 to 4,
**characterised in that**
the granulate acts as a carrier for cosmetic active ingredients and/or auxiliary substances.

6. Cosmetic composition containing a granulate based on pyrogenically-produced silicon dioxide and at least one constituent selected from:
Absorbents, astringents, antimicrobial substances, antioxidants, anti-perspirants, anti-foam agents, anti-dandruff active ingredients, antistatic agents, binders, biological additives, bleaching agents, chelating agents, deodorants, emolients, emulsifiers, emulsion stabilisers, depilatory agents, colourants, moisture-containing agents, film-formers, perfumes, flavourings, hair colourants, preservatives, anti-corrosion agents, cosmetic oils, solvents, mouth care products, oxidation agents, vegetable constituents, buffering substances, reducing agents, abrasives, detergents, propellants, opacity agents, UV-filters and absorbers, denaturing agents, viscosity regulators and vitamins **characterised in that** the granulate has an average grain diameter of 10 to
120 µm and a BET-surface of 40 to 400 m²/g (determined to DIN 66 131 with nitrogen)

7. Adsorbate from a granulate based on pyrogenically-produced silicon dioxide and at least one other substance, selected from cosmetic active ingredients and auxiliary agents
**characterised in that**
the granulate has an average grain size of 10 to 120 µm and a BET-surface of 40 to 400 m²/g (determined to DIN 66 131 with nitrogen).

8. Adsorbate according to claim 7, **characterised in that**
the cosmetic active ingredients and auxiliary substances are selected from the substances listed in claim 6.

9. Process for the production of an adsorbate according to one of claims 7 to 8 comprising:
(a) melting of the substance(s) to be adsorbed, selected from cosmetic active ingredients and auxiliaries, or distribution of these in a solvent;
(b) mixing of the granulate based on pyrogenically-produced silicon dioxide with the mixture from step (a); and
(c) optionally, removal of the solvent.

## Patentansprüche

1. Verwendung eines Granulats auf Basis von pyrogen hergestelltem Siliciumdioxid in einer kosmetischen Zusammensetzung, **dadurch gekennzeichnet, dass** das Granulat einen mittleren Korndurchmesser von 10 bis 120 µm und eine BET-Oberfläche von 40 bis 400 m²/g (Bestimmung nach DIN 66 131 mit Stickstoff) aufweist.

2. Verwendung nach Anspruch 1, **dadurch gekennzeichnet, dass** die kosmetische Zusammensetzung in Form eines Pulvers, einer Flüssigkeit, eines Schaums, eines Sprays, eines Gels, einer Creme, einer Salbe, einer Paste, eines Stifts oder einer Tablette vorliegt.

3. Verwendung nach Anspruch 1 oder 2, **dadurch gekennzeichnet, dass** es sich bei der kosmetischen Zusammensetzung um eine Seife, ein Syndet, ein flüssiges Wasch- oder Duschpräparat, einen Badezusatz, ein Make-up-Entfernungsmittel, ein Peeling-Präparat, eine Hautcreme, eine Hautlotion, eine Gesichtsmaske, ein Fußpflegemittel, ein Sonnenschutzmittel, ein Hautbräunungsmittel, ein Depigmentierungsmittel, ein Insekten abwehrendes Mittel, ein Nassrasurmittel, ein Pre-Shave-Präparat, ein After-Shave-Pflegemittel, ein Haarentfernungsmittel, eine Zahncreme, ein Haarshampoo, ein Haarpflegemittel, ein Dauerwellenmittel, ein Glättungsmittel, ein Frisurfestigungsmittel, ein Haarfarbänderungsmittel, ein Deodorant, ein Antitranspirationsmittel, ein Gesichts-Make-up, ein Augen-Make-up, ein Lippenpflegemittel, ein dekoratives Lippenpflegemittel oder ein Nagelpflegemittel handelt.

4. Verwendung nach Anspruch 3, **dadurch gekennzeichnet, dass** es sich bei dem Nassrasurmittel um einen Stift, eine Creme, ein Gel oder einen Schaum handelt;
bei dem Haarpflegemittel um eine Haarkur, eine Spülung oder einen Conditioner handelt;
bei dem Frisurfestigungsmittel um einen Haarfestiger, ein Haarspray, einen Haarlack, ein Haargel oder ein Haarwachs handelt;
bei dem Haarfarbänderungsmittel um ein Blondiermittel, ein Haarfärbemittel, eine Tönung oder einen Farbfestiger handelt;
bei dem Deodorant oder Antitranspirationsmittel um einen Stift, ein Roll-on, eine Lotion, ein Puder oder ein Spray handelt;
bei dem Gesichts-Make-up um eine getönte Tagescreme, eine Pudercreme, ein Gesichtspuder, ein Creme-Make-up oder ein Rouge handelt;
bei dem Augen-Make-up um einen Lidschatten, eine Wimperntusche, einen Kajalstift, einen Eyeliner oder einen Augenbrauenstift handelt;
bei dem dekorativen Lippenpflegemittel um einen Lippenstift, ein Lipgloss oder einen Lippenkonturenstift handelt und
bei dem Nagelpflegemittel um einen Nagellack, einen Nagellackentferner, einen Nagelhautentferner, einen Nagelhärter oder eine Nagelpflegecreme handelt.

5. Verwendung nach einem der Ansprüche 1 bis 4, **dadurch gekennzeichnet, dass** das Granulat als Träger für kosmetische Wirk- und/oder Hilfsstoffe wirkt.

6. Kosmetische Zusammensetzung, enthaltend ein Granulat auf Basis von pyrogen hergestelltem Siliciumdioxid und mindestens einen Bestandteil ausgewählt aus:
Absorptionsmitteln, Adstringenzien, antimikrobiellen Stoffen, Antioxidantien, Antiperspirantien, Antischaummitteln, Antischuppenwirkstoffen, Antistatika, Bindemitteln, biologischen Zusatzstoffen, Bleichmitteln, Chelatbildnern, Desodorierungsmitteln, Emollentien, Emulgatoren, Emulsionsstabilisatoren, Enthaarungsmitteln, Farbstoffen, Feuchthaltemitteln, Filmbildnern, Geruchsstoffen, Geschmacksstoffen, Haarfärbemitteln, Konservierungsstoffen, Korrosionsschutzmitteln, kosmetischen Ölen, Lösungsmitteln, Mundpflegestoffen, Oxidationsmitteln, pflanzlichen Bestandteilen, Puffersubstanzen, Reduktionsmitteln, Schleifmitteln, Tensiden, Treibgasen, Trübungsmitteln, UV-Filtern und -Absorbern, Vergällungsmitteln, Viskositätsreglern und Vitaminen, **dadurch gekennzeichnet, dass** das Granulat einen mittleren Korndurchmesser von 10 bis 120 µm und eine BET-Oberfläche von 40 bis 400 m²/g (Bestimmung nach DIN 66 131 mit Stickstoff) aufweist.

7. Adsorbat aus einem Granulat auf Basis von pyrogen hergestelltem Siliciumdioxid und mindestens einer weiteren Substanz, ausgewählt aus kosmetischen Wirk- und Hilfsstoffen, **dadurch gekennzeichnet, dass** das Granulat einen mittleren Korndurchmesser von 10 bis 120 µm und eine BET-Oberfläche von 40 bis 400 m²/g (Bestimmung nach DIN 66 131 mit Stickstoff) aufweist.

8. Adsorbat nach Anspruch 7, **dadurch gekennzeichnet, dass** die kosmetischen Wirk- und Hilfsstoffe aus den in Anspruch 6 genannten Stoffen ausgewählt sind.

9. Verfahren zur Herstellung eines Adsorbats gemäß einem der Ansprüche 7 bis 8, umfassend:
(a) Schmelzen der zu adsorbierenden Substanz(en), ausgewählt aus kosmetischen Wirk- und Hilfsstoffen, oder Verteilen derselben in einem Lösemittel;
(b) Mischen des Granulats auf Basis von pyrogen hergestelltem Siliciumdioxid mit der Mischung aus Schritt (a); und
(c) gegebenenfalls Entfernen des Lösemittels.

## Revendications

1. Utilisation d'un granulat à base de dioxyde de silicium produit par voie pyrogène dans une composition cosmétique, **caractérisée en ce que** le granulat présente un diamètre moyen de particule de 10 à 120 µm et une surface BET de 40 à 400 m²/g (déterminée selon la norme DIN 66 131 avec de l'azote).

2. Utilisation selon la revendication 1, **caractérisée en ce que** la composition cosmétique est sous forme de poudre, de liquide, de mousse, de spray, de gel, de crème, de baume, de pâte, de bâton ou de comprimé.

3. Utilisation selon l'une quelconque des revendications 1 et 2, **caractérisée en ce que** la composition cosmétique est un savon, un savon synthétique "sans savon", une préparation liquide de lavage ou de douche, un additif pour le bain, un démaquillant, un produit d'exfoliation, une crème pour la peau, une lotion pour la peau, un masque facial, un produit de soin pour les pieds, une crème de protection solaire, un produit de bronzage de la peau, un produit de dépigmentation, un répulsif d'insectes, un produit de rasage humide, un produit de prérasage, un produit de soin après rasage, un produit dépilatoire, un dentifrice, un shampooing pour cheveux, un produit de soin pour cheveux, un produit pour permanente, un produit lissant, un produit de coiffage des cheveux, un produit de coloration des cheveux, un déodorant, un anti-perspirant, un produit de maquillage pour le visage, un produit de maquillage pour les yeux, un produit de soin des lèvres, un produit de soin des lèvres décoratif ou un produit de soin des ongles.

4. Utilisation selon la revendication 3, **caractérisée en ce que** le produit de rasage humide est un bâton, une crème, un gel ou une mousse ; le produit de soin de cheveux est un masque pour cheveux, un après-shampooing ou un produit de soin ;
le produit de coiffage des cheveux est une lotion pour mise en plis, un spray, une laque, un gel ou une cire pour cheveux ;
le produit de coloration des cheveux est un agent de décoloration, un agent de coloration pour cheveux, une teinture ou un agent de fixation de la couleur ;
le déodorant ou l'anti-perspirant est un bâton, un déodorant à bille, une lotion, une poudre ou un spray ; le produit de maquillage pour le visage est une crème de jour teintée, un fond de teint crème-à-poudre, une poudre pour le visage, un fond de teint en crème ou un fard à joues ;
le produit de maquillage pour les yeux est une ombre à paupières, un mascara, un crayon khôl, un eye-liner ou un crayon à sourcils ;
le produit de soin des lèvres décoratif est un rouge à lèvres, un brillant à lèvres ou un crayon à lèvres et
le produit des soins pour les ongles est un vernis à ongle, un dissolvant de vernis à ongle, un produit d'élimination des cuticules, un durcisseur pour ongles ou une crème de soin des ongles.

5. Utilisation selon l'une quelconque des revendications 1 à 4, **caractérisée en ce que** le granulat agit comme support pour des ingrédients actifs et/ou des substances auxiliaires cosmétiques.

6. Composition cosmétique contenant un granulat à base de dioxyde de silicium produit par voie pyrogène et d'au moins un constituant choisi parmi :
les absorbants, les astringents, les substances antimicrobiennes, les antioxydants, les anti-perspirants, les antimousses, les ingrédients actifs antipelliculaires, les agents antistatiques, les liants, les additifs biologiques, les agents de décoloration, les chélatants, les déodorants, les émollients, les émulsifiants, les stabilisateurs d'émulsions, les agents dépilatoires, les colorants, les agents contenant de l'humidité, les agents de formation de film, les parfums, les aromes, les colorants pour cheveux, les conservateurs, les agents anti-corrosion, les huiles cosmétiques, les solvants, les produits de soin buccaux, les agents d'oxydation, les constituants végétaux, les substances tampon, les réducteurs, les abrasifs, les détergents, les agents propulseurs, les agents opacifiants, les filtres et absorbants des UV, les agents de dénaturation, les régulateurs de la viscosité et les vitamines, **caractérisée en ce que** le granulat présente un diamètre moyen de particule de 10 à 120 µm et une surface BET de 40 à 400 m²/g (déterminée selon la norme DIN 66 131 avec de l'azote).

7. Produit adsorbé constitué par un granulat à base de dioxyde de silicium produit par voie pyrogène et d'au moins une autre substance, choisie parmi les ingrédients actifs et les agents auxiliaires cosmétiques, **caractérisé en ce que** le granulat présente une taille moyenne de particule de 10 à 120 µm et une surface BET de 40 à 400 m²/g (déterminée selon la norme DIN 66 131 avec de l'azote).

8. Produit adsorbé selon la revendication 7, **caractérisé en ce que** les ingrédients actifs et les substances auxiliaires cosmétiques sont choisis parmi les substances listées dans la revendication 6.

9. Procédé pour la production d'un produit adsorbé selon l'une quelconque des revendications 7 à 8, constitué par :
(a) la fusion de la ou des substances à adsorber, choisies parmi les ingrédients actifs et les auxiliaires cosmétiques, ou leur distribution dans un solvant ;
(b) le mélange du granulat à base de dioxyde de silicium produit par voie pyrogène avec le mélange de l'étape (a) ; et
(c) éventuellement, l'élimination du solvant.
